# EUROPÄISCHE PATENTSCHRIFT

(11) **EP 2 220 508 B1**
(45) Veröffentlichungstag und Bekanntmachung des Hinweises auf die Patenterteilung: **26.12.2012**
(21) Anmeldenummer: 08861702.2
(22) Anmeldetag: 17.11.2008
(51) Int. Cl.: G01N 33/68

(54) **VERFAHREN ZUR EXTRAKTION VON MEMBRANPROTEINEN**
METHOD FOR THE EXTRACTION OF MEMBRANE PROTEINS
PROCÉDÉ D'EXTRACTION DE PROTÉINES MEMBRANAIRES

(30) Priorität: 15.12.2007 DE 102007060599
(43) Veröffentlichungstag der Anmeldung: 25.08.2010
(73) Patentinhaber: Merck Patent GmbH, 64293 Darmstadt (DE)
(72) Erfinder: VON HAGEN, Joerg, 64319 Pfungstadt (DE); MICHELSEN, Uwe, 69469 Weinheim (DE)
(86) Internationale Anmeldenummer: PCT/EP2008/009704
(87) Internationale Veröffentlichungsnummer: WO 2009/077046

(56) Entgegenhaltungen:
- WO-A-2005/047310
- WO-A-2005/103303
- WO-A-2007/011959
- WO-A-2007/083154

## Beschreibung

Die vorliegende Erfindung betrifft die Verwendung von linearen amphipathischen Kohlenhydrat-Polymeren zur Extraktion von Membranproteinen aus biologischen Proben, Verfahren zur Extraktion von Membranproteinen, ein Kit zur Extraktion dieser Proteine sowie dessen Verwendung.

Der Nachweis beziehungsweise die Analyse von Proteinen, ganz besonders von Membranproteinen, gewinnt in der Medizin zunehmende Bedeutung. Die Mehrheit der in der pharmazeutischen Forschung untersuchten Systeme beinhalten Membranproteine. Membranproteinen kommt bei einer Reihe biologischer Funktionen eine besondere Bedeutung zu. So spielen viele Membranproteine bei der Entstehung von Krankheiten eine große Rolle, so dass das Verständnis ihrer Funktion bei Entwicklung von Medikamenten zunehmende Wichtigkeit erlangt. Daher ist die Information über die strukturelle Eigenschaft und über die Funktion dieser Proteine Grundlage für das Verständnis der Mechanismen.

Membranproteine, insbesondere Transmembranproteine, besitzen hydrophobe Regionen und sind damit in Membranen verankert und somit schwer löslich in Wasser. Um eine in-vitro-Analyse der Proteine zu ermöglichen, werden Membranproteine üblicherweise durch Zusatz von Detergentien solubilisiert. Die Isolierung von Membranproteinen mit Detergentien hat aber den gravierenden Nachteil, dass die native Struktur der Proteine durch den Einfluss des Detergens denaturiert wird. Gängige Detergentien sind entweder ionischer Natur, wie beispielsweise Natriumdodecylsulfat (SDS), oder nicht-ionischer Natur, wie beispielsweise Triton-X 100. Der Einsatz von SDS führt zu einer vollständigen Denaturierung aller Proteine und damit auch der Membranproteine, das heißt strukturelle und funktionelle Untersuchungen an den Membranproteinen sind nicht oder nur sehr stark eingeschränkt möglich. Triton-X 100 ist nur in Ausnahmefällen in der Lage Membranproteine, insbesondere multiple transmembranöse Proteine, effektiv zu extrahieren, so dass die gewünschten Untersuchungen gar nicht erst erfolgen können. Ein weiterer Nachteil von Detergenzien wie Triton-X 100 und anderen besteht darin, dass diese Reagenzien nicht direkt kompatibel mit weiteren Analysetechniken (z.B. Massenspektrometrie) sind.

Es bestand daher die Aufgabe, Verfahren bereit zu stellen, mit deren Hilfe Membranproteine aus biologischen Proben extrahiert werden können und direkt mit nachfolgenden Analysetechniken untersucht werden können.

Es wurde nun gefunden, dass in Wasser unlösliche, lineare amphipathische Kohlenhydrat-Polymere, die für die Protein-Stabilisierung und Protein-Aufreinigung bekannt sind, unerwartet gut zur Extraktion von Membranproteinen insbesondere aus komplexen biologischen Proben geeignet sind.

Gegenstand der vorliegenden Erfindung ist demgemäss die Verwendung von linearen amphipathischen Kohlenhydrat-Polymeren zur Extraktion von Membranproteinen aus biologischen Proben, da diese Art Polymer in wässrigen Puffersystemen nicht löslich ist und nach erfolgter Extraktion mechanisch (z.B. durch Zentrifugation, Filtrieren, u.ä.) entfernt werden kann.

In einer bevorzugten Ausführungsform wird zur Extraktion eine wässrige Lösung enthaltend zwischen 0,5 und 10 Gew% an linearen amphipathischen Kohlenhydrat-Polymeren eingesetzt.

In einer weiteren bevorzugten Ausführungsform handelt es sich bei den Membranproteinen um Proteine, die eine oder mehr Transmembranpassagen aufweisen.

In einer weiteren bevorzugten Ausführungsform handelt es sich bei den biologischen Proben um Gewebe, Zellen, Zellkulturen, Körperflüssigkeiten, Bakterien, Pilzen, Viren und/oder Pflanzen.

Gegenstand der vorliegenden Erfindung ist auch ein Verfahren zur Extraktion von Membranproteinen aus bevorzugt nativen, biologischen Proben, dadurch gekennzeichnet, dass eine bevorzugt native, biologische Probe mit einer Mischung zumindest enthaltend ein oder mehrere lineare amphipathische Kohlenhydrat-Polymere, gegebenenfalls unter mechanischer Einwirkung, versetzt wird. In der Mischung liegt das Kohlenhydrat-Polymer vollständig oder nahezu vollständig suspendiert, d.h. ungelöst vor. Bevorzugt liegt für das erfindungsgemäße Verfahren das Kohlenhydrat-Polymer in Suspension, d.h. vollständig ungelöst, vor.

In einer bevorzugten Ausführungsform wird die biologische Probe vorher lysiert.

In einer besonders bevorzugten Ausführungsform erfolgt das Lysieren der biologischen Proben durch Zusatz von Detergenzien, oberflächenaktiven Substanzen und/oder Porenbildnern.

In einer bevorzugten Ausführungsform erfolgt die mechanische Einwirkung durch Schütteln oder Rühren.

In einer weiteren bevorzugten Ausführungsform sind die linearen amphipathischen Kohlenhydrat-Polymere ungeladen.

In einer bevorzugten Ausführungsform bestehen die linearen amphipathischen Kohlenhydrat-Polymere aus Inulin oder Derivaten davon. In einer weiteren bevorzugten Ausführungsform haben die linearen amphipathischen Kohlenhydrat-Polymere ein lineares Poly-Fructose-Rückgrat.

In einer weiteren bevorzugten Ausführungsform erfolgt die Extraktion bei Temperaturen zwischen 4 und 37°C.

In einer weiteren bevorzugten Ausführungsform beträgt die Konzentration der linearen amphipathischen Kohlenhydrat-Polymere in der Extraktionslösung zwischen 0.5 und 10 Gew%.-%.

In einer weiteren bevorzugten Ausführungsform werden die ein oder mehreren linearen amphipathischen Kohlenhydrat-Polymere nach der Extraktion mittels Zentrifugation, Filtration, magnetischer Separation, Sedimentation oder chromatographischer Methoden entfernt, so dass in der resultierenden Lösung die extrahierten Proteine zurückbleiben und ohne störende Einflüsse des Extraktionsmittels (=lineare amphipathische Kohlenhydrat-Polymere) weiterer Analysen unterzogen werden können. In einer weiteren bevorzugten Ausführungsform werden in dem erfindungsgemäßen Verfahren die ein oder mehreren linearen amphipathischen Kohlenhydrat-Polymere als Beschichtung auf magnetischen Partikeln eingesetzt.

Gegenstand der vorliegenden Erfindung ist auch die Verwendung eines Kits zur Extraktion von Membranproteinen nach dem erfindungsgemäßen Verfahren. Der Kit enthält zumindest ein oder mehrere lineare amphipathische Kohlenhydrat-Polymere als Feststoff oder in Flüssigkeit sowie mindestens ein Lysemittel ausgewählt aus der Gruppe der Detergenzien, oberflächenaktiven Substanzen und/oder Porenbildner. Kern der vorliegenden Erfindung ist, dass das erfindungsgemäße Verfahren bzw. der erfindungsgemäße Kit geeignet sind, Membranproteine, insbesondere mehrfach membrangängige Proteine, schonend und möglichst strukturerhaltend zu extrahieren. Dem Fachmann ist bekannt, dass insbesondere bei mehrfach membrangängigen Proteinen eine funktionserhaltende Extraktion aus der Membran kaum möglich ist, da sich die 3-D Struktur des Proteins nach der Extraktion aus der Membran zwangsläufig ändert, wenn die Transmembrandomänen aus der hydrophoben Umgebung der Membran entfernt werden. Daher bedeutet im Hinblick auf die erfindungsgemäße Extraktion von mehrfach membrangängigen Proteinen, der Begriff "nativ", dass die extrahierten Membranproteine zwar in der Regel nicht funktionserhaltend, wohl aber schonend und möglichst strukturerhaltend extrahiert werden. Beispielsweise ermöglicht die erfindungsgemäße Extraktion die massenspektrometrische und immunologische Messung insbesondere der Transmembrandomänen der Proteine. Das ist mit herkömmlichen Methoden wie z.B. der Extraktion mit Triton-X100, Nonidet P40 oder anderen Detergenzien die standardmäßig Anwendung finden in Bezug auf die Proteinausbeute und Funktionserhalt der zu untersuchenden Proteine nicht vergleichbar möglich, insbesondere nicht, wenn der Extrakt ohne Aufreinigung oder Entfernung des Zusatzes direkt weiter analysiert werden soll.

Bei Membranproteinen, die nur mit einem für ihre Funktion bzw. Aktivität irrelevanten Teil in der Membran verankert sind, wie z.B. GPI-Anker-Proteine, bedeutet die erfindungsgemässe "native" Extraktion, dass das Protein weitgehend struktur- und aktivitätserhaltend extrahiert werden kann. Beispielsweise können in diesem Fall zum Nachweis des Proteins entsprechende Aktivitätsmessungen durchgeführt werden.

Native Proben sind Proben, in denen die zu extrahierenden Membranproteine noch weitgehend in ihrer nativen, d.h. in der für ihre natürliche Funktion notwendigen Konformation vorliegen, bzw. Proben, in denen die Membranproteine noch Aktivität zeigen.

Die Extraktion von Membranproteinen gemäß der vorliegenden Erfindung. kann aus allen dem Fachmann bekannten biologischen Proben erfolgen. Biologische Proben sind erfindungsgemäß alle Proben, in denen die Membranproteine in einer natürlichen Membran eingebunden vorliegen. Vorzugsweise handelt es sich bei den biologischen Proben um Gewebe, wie z.B. Biopsien und histologische Präparate, Zellen, Zellkulturen und/oder zellhaltige Körperflüssigkeiten, wie z.B. Blut, Urin, Liquor oder Speichel, sowie Bakterien, Pflanzen und/oder Pilze. Auch Membranproteine aus membranhaltigen Zellkompartimenten oder Zellfragmenten können erfindungsgemäß extrahiert werden. Die Extraktion von Proteinen aus Geweben und Zellkulturen erlaubt insbesondere den Nachweis spezifischer Proteine, z.B. den Nachweis von Proteinen, die auf das Vorhandensein von Krankheiten schließen lassen. Daher ist das erfindungsgemäße Verfahren insbesondere auch für pathologisch interessante Gewebeproben vorteilhaft.

Das erfindungsgemäße Verfahren ist insbesondere geeignet für Transmembranproteine und in ganz besonderer Weise für mehrfach membrangängige Proteine, d.h. Proteine, die zwei oder mehr Transmembranpassagen aufweisen, insbesondere multihelikale Transmembranproteine, wie z.B. heptahelikale Transmembranproteine. Die Klasse der heptahelikalen Transmembranproteine umfasst derzeit ca. 250 bekannte Proteine. Die Transmembranproteine können hierbei in folgende Subklassen eingeteilt werden:
- Class A Rhodopsine, Hormonproteine, (Rhod)opsin, Olfactory, Prostanoide, Nucleotid-Analoga, Cannabinoid, Platelet activating factor, Gonadotropin-freisetzende Hormone, Thyrotropin-freisetzende Hormone und Secretagogue, Melatonin, virale Proteine, Lysosphingolipid & LPA (EDG), Leukotriene B4 Rezeptoren, Class A Orphan und andere,
- Class B Secretine, beispielsweise Calcitonin, Corticotropin releasing factor, Gastric inhibitory peptide, Glucagon, Growth hormone-releasing Hormone, Parathyroid Hormone, PACAP, Secretin, Vasoactive intestinale Polypeptide, Diuretic Hormone, EMR1, Latrophilin, Brainspecific angiogenesis inhibitor (BAI), Methuselah-like proteins (MTH), Cadherin EGF LAG (CELSR), Very large G-protein coupled Rezeptoren
- Class C Metabotropic glutamate/pheromone, beispielsweise Metabotropic glutamate, Calcium-sensing like, Putative pheromone receptors, GABA-B, Orphan GPRC5, Orphan GPCR6, Bride of sevenless proteins (BOSS), Taste receptors (T1R),
- Class D Fungal pheromone, beispielsweise Fungal pheromone A-Factor like (STE2,STE3), Fungal pheromone B like (BAR,BBR,RCB,PRA), Fungal pheromone M- and P-Factor, Class E cAMP receptors, Frizzled/Smoothened family, frizzled, Smoothened und in die folgenden Non-GPCR-Familien: Class Z Archaeal/bacterial/fungal opsins.

Die erfindungsgemäß eingesetzten linearen amphipathischen Kohlenhydrat-Polymere können linear oder leicht verzweigt sein. Das bedeutet, dass leicht verzweigte Kohlenhydratpolymere mit 1 oder 2 Verzweigungsstellen pro Molekül auch noch als lineare Kohlenhydrat-Polymere im Sinne der vorliegenden Erfindung verstanden werden.

Die erfindungsgemäß eingesetzten linearen amphipathischen Kohlenhydrat-Polymere sind typischerweise ganz oder zumindest vorwiegend in Wasser unlöslich. Dadurch können sie nach erfolgter Extraktion mittels einfacher Methoden wie Filtration, Zentrifugation etc. abgetrennt werden.

Erfindungsgemäß geeignete lineare amphipathische Kohlenhydrat-Polymere sind beispielsweise bekannt aus WO 2005/047310.

Es handelt sich bevorzugt um Fructane beziehungsweise Fructan-Derivate. Fructane zeichnen sich dadurch aus, dass an ein Saccharosemolekül ein oder mehrere Fructosemoleküle gebunden sind.

Je nach der Bindungsstelle des Fructosylrests an die Saccharose unterscheidet man drei Fructan-Grundtypen:
1-Kestosen: Beim Inulin sind die die Fructosylreste über β-2,1-Bindungen an den Fructosylrest der Saccharose verknüpft. Das einfachste Inulin ist die 1-Kestotriose oder Isokestose.
6-Kestosen: Ist der Fructosylrest über eine β-2,6-Bindung an den Fructosylrest der Saccharose verknüpft, spricht man von 6-Kestotriose oder Kestose. Fruktane dieses Typs bezeichnet man manchmal als Laevane oder Phleine.
Neokestosen: Bei den Neokestosen oder 6G-Kestosen hängt der Fructosylrest am C6 des Glucosylrests der Saccharose.

Genauso können die erfindungsgemäß eingesetzten Fructane oder Fructan-Derivate verschiedene Verknüpfungsmuster in einem Molekül aufweisen und somit eine Mischung aus zwei oder mehreren Grundtypen darstellen.

Besonders bevorzugt werden erfindungsgemäß Inulin oder Inulin-Derivate eingesetzt. Weitere Informationen zu Inulin finden sich beispielsweise in WO 2005/047310, Seite 3, Zeile 4 bis Seite 5, Zeile 9.

Geeignetes Inulin oder Inulin-Derivate weisen einen Polymerisationsgrad zwischen 3 und 500, bevorzugt zwischen 3 und 100, besonders bevorzugt zwischen 10 und 50 auf. Besonders bevorzugt sind Inulin-Derivate mit einem Polymerisationsgrad von 20, 21, 22, 23, 24, 25, 26, 27, 28, 29 oder 30.

Bevorzugt tragen die erfindungsgemäß eingesetzten linearen amphipathischen Kohlenhydrat-Polymere keine Ladung, d.h. sie sind nicht mit geladenen Gruppen derivatisiert.

Bevorzugt werden als lineare amphipathische Kohlenhydrat-Polymere erfindungsgemäß lineare Kohlenhydrat-Polymere eingesetzt, die ein oder mehrfach mit hydrophoben, insbesondere C3 bis C18 Alkylketten derivatisiert sind. Besonders bevorzugt bestehen dabei die Kohlenhydrat-Polymere aus Inulin.

Erfindungsgemäß bevorzugte Inulin-Derivate sind Verbindungen der Formel I:

G(O-CO-NH-R¹)ₐ-[F(O-CO-NH-R²)_{b}]ₙ I

worin
G eine terminale Glucosylgruppe darstellt, in der eine oder mehrere Hydroxylgruppen unabhängig voneinander mit einer Gruppe der Formel (O-CO-NH-R¹) derivatisiert sein können,
R¹ ein ungeladener Rest, insbesondere ein linearer oder verzweigter gesättigter oder ungesättigter Kohlenwasserstoffrest mit 1 bis 25 Kohlenstoffatomen, ist.
a eine Zahl zwischen 1 und 4 ist,
F ein Fructosylrest ist, in dem eine oder mehrere Hydroxylgruppen unabhängig voneinander mit einer Gruppe der Formel (O-CO-NH-R²) derivatisiert sein können,
R² ein ungeladener Rest, insbesondere ein linearer oder verzweigter gesättigter oder ungesättigter Kohlenwasserstoffrest mit 1 bis 25 Kohlenstoffatomen, ist,
b eine Zahl zwischen 1 und 3 ist für Fructosylreste innerhalb der Kette und eine Zahl zwischen 1 und 4 ist für endständige Fructosylreste,
n eine Zahl ist zwischen 3 und 500, bevorzugt zwischen 3 und 100, besonders bevorzugt zwischen 10 und 50, insbesondere 20, 21, 22, 23, 24, 25, 26, 27, 28, 29 oder 30. Besonders bevorzugt ist n = 24.

Der durchschnittliche Derivatisierungsgrad pro Glucolsyl- bzw. Fructosyl-Einheit liegt zwischen 0,02 und 3, bevorzugt zwischen 0,05 und 1, besonders bevorzugt zwischen 0,05 und 0,5.

In einer bevorzugten Ausführungsform sind die Gruppen R¹ und/oder R² unabhängig voneinander Alkyl-, Alkenyl- oder Alkinylgruppen mit 1 bis 25 Kohlenstoffatomen, bevorzugt 3 bis 22, besonders bevorzugt 3 bis 18 Kohlenstoffatomen, beispielsweise n-octyl-, n-decyl-, n-octadecyl.

Unter dem Begriff lineare amphipathische Kohlenhydrat-Polymere wird erfindungsgemäß eine einzige Sorte der linearen amphipathischen Kohlenhydrat-Polymere verstanden oder eine Mischung aus verschiedenen linearen amphipathischen Kohlenhydrat-Polymeren.

In einer besonders bevorzugten Ausführungsform handelt es sich bei dem erfindungsgemäß eingesetzten linearen amphipathischen Kohlenhydrat-Polymer um Nvoy Polymer, ein von der Firma Novexin (Cambridge, GB) käuflich zu erwerbendes lineares amphipathisches Kohlenhydratpolymer bestehend aus ungeladenen Molekülen mit einem Molekulargewicht von ca. 5 kD, die aus linearen Poly-Fructose-Einheiten bestehen, die hydrophob derivatisiert sind.

Gegenstand der vorliegenden Erfindung ist ein Verfahren zur Extraktion von Membranproteinen aus biologischen Proben, wobei eine biologische Probe zumindest mit einem oder mehreren linearen amphipathischen Kohlenhydrat-Polymeren, gegebenenfalls unter mechanischer Einwirkung, versetzt und anschließend separiert werden kann.

Typischerweise wird die biologische Probe dabei nicht mit mindestens einem in fester Form vorliegenden linearen amphipathischen Kohlenhydrat-Polymer versetzt sondern mit einer wässrigen Lösung, in der zumindest ein lineares amphipathisches Kohlenhydrat-Polymer enthalten ist. Die linearen amphipathischen Kohlenhydrat-Polymere sind dabei typischerweise in der Suspension als Aufschlämmung enthalten. Die wässrige Suspension zumindest enthaltend ein oder mehrere lineare amphipathische Kohlenhydrat-Polymere wird erfindungsgemäß auch als Extraktionslösung bezeichnet.

Als wässrige Lösung werden typischerweise Wasser oder wässrige Puffersysteme verwendet. Genauso können als wässrige Lösung erfindungsgemäß Wasser bzw. wässrige Puffersysteme eingesetzt werden, die bis zu 20 Volumenprozent eines oder mehrerer mit Wasser mischbarer Lösungsmittel enthalten.

In einer bevorzugten Ausführungsform wird ein wässriges Puffersystem verwendet. Dieses Puffersystem sollte einen pH-Bereich zwischen 4,5 und 9.0 aufweisen, bevorzugt zwischen 6,5 und 8,0. Besonders bevorzugt liegt der pH-Wert der Lösung zwischen pH 7,0 und 7,5.

Geeignete Puffer sind alle Puffersysteme, die physiologische Bedingungen erzeugen, d.h. Proteine nicht denaturieren. Beispiele sind PIPES, HEPES, Phosphatpuffer und Tris basierte Puffer.

Typischerweise liegt das lineare amphipathische Kohlenhydrat-Polymer in der wässrigen Lösung in einer Konzentration zwischen 0,5 und 10 Gew%, bevorzugt zwischen 0,5 und 5 Gew%, besonders bevorzugt zwischen 1 und 3 Gew.% vor.

In der einfachsten Ausführungsform der vorliegenden Erfindung wird die biologische Probe mit einer wässrigen Suspension zumindest enthaltend ein oder mehrere lineare amphipathische Kohlenhydrat-Polymere, versetzt. Vorzugsweise erfolgt das genannte Verfahren unter mechanischer Einwirkung, beispielsweise durch Schütteln oder Rühren. Auf diese Weise wird die Extraktion der Membranproteine beschleunigt und die Ausbeute an extrahierten Proteinen verbessert.

In einer weiteren Ausführungsform des erfindungsgemäßen Verfahrens kann die biologische Probe zunächst lysiert werden, d.h. die zelluläre Grundstruktur wird vor Anwendung des erfindungsgemäßen Verfahrens zerstört. Diese Vorbehandlung kann auf alle dem Fachmann bekannten Arten erfolgen, z.B. durch manuelle Homogenisierung oder mechanisches Schütteln.

Insbesondere kann das Lysieren der biologischen Proben auch durch Zusatz von dem Fachmann bekannten Detergenzien, oberflächenaktiven Substanzen und/oder Porenbildner erfolgen. Durch das vorherige Lysieren der Probe wird das Ergebnis der Extraktion hinsichtlich der Ausbeute an erhaltenen Membranproteinen nochmals verbessert. So verbleiben beim Lysieren die Membranproteine in der Membran, die Membranen bzw. Membranfragmente können aber in einfacher Weise von den anderen Zellbestandteilen abgetrennt werden. Bevorzugt erfolgt die Lyse mit Digitonin.

Die Lyse kann auch gleichzeitig mit der Extraktion durchgeführt werden, dann erhält man jedoch komplexere Proteingemische.

Die wässrige Suspension zumindest enthaltend ein oder mehrere lineare amphipathische Kohlenhydrat-Polymere zum Einsatz in dem erfindungsgemäßen Verfahren kann zusätzliche Additive und Hilfsstoffe enthalten. Entsprechende Additive und Hilfsstoffe sind dem Fachmann bekannt und umfassen beispielsweise Detergenzien, oberflächenaktive Substanzen, Porenbildner, biologische bzw. physiologische Puffersysteme, Stabilisatoren, Mineralsalze und/oder Inhibitoren (beispielsweise Proteaseinhibitoren).

Die erfindungsgemäßen Verfahren können bei Temperaturen oberhalb von 0°C durchgeführt werden, typischerweise zwischen 0 und 95°C. Wenn insbesondere hohe Proteinausbeuten erzielt werden sollen und der Erhalt von Aktivität oder Struktur sekundär sind, kann die Extraktion bei hohen Temperaturen (über 37°C) durchgeführt werden. Derartige Extraktionen können besonders gut für Western Blot Analysen genutzt werden. Vorzugsweise erfolgt die erfindungsgemäße Extraktion bei 0 bis 37°C, besonders bevorzugt zwischen 0 und 8 °C, insbesondere zwischen 0 und 4°C. Bei den bevorzugten Temperaturen wird eine verbesserte Extraktion in relativ kurzer Zeit und die Beibehaltung der Proteinaktivität des Proteins beobachtet. Zur schonenden Extraktion, insbesondere von empfindlicheren Membranproteinen empfiehlt sich die Durchführung des erfindungsgemäßen Verfahrens bei tieferen Temperaturen innerhalb des angegebenen Temperaturintervalls, unter Berücksichtigung einer dafür erforderlichen längeren Extraktionszeit.

Typische Extraktionszeiten liegen zwischen 30 Minuten und 16 Stunden. Wenn aktive Proteine extrahiert werden sollen, sollte der pH-Wert der Extraktionslösung bevorzugt bei ca. pH 7.4 liegen, ansonsten können auch Extraktionslösungen mit pH-Werten zwischen 2 und 10 eingesetzt werden.

Die erfindungsgemäß extrahierten Proteine können beispielsweise für massenspektrometrische Untersuchungen (z.B. Maldi, Esi und Seldi) direkt eingesetzt werden oder auch mittels allen anderen dem Fachmann bekannten Arten von Proteinanalytik untersucht werden, z.B. mittels Elektrophorese (z.B. Gelelektrophorese, insbesondere auch 2-dimensionale Gelelektrophorese), immunchemischer Nachweismethoden (z.B. Western Blot Analyse, ELISA, RIA), Protein-Arrays (z.B planare und beadbasierende Systeme), sowie aller chromatographischer Trennverfahren, insbesondere biochromatographischer Trennverfahren (IEX, SEC, HIC, Affinitätschromatographie und Hydrophobe Interaktionschromatographie) oder in Aktivitätsassays eingesetzt werden. Insbesondere eignen sich zur Analyse der erfindungsgemäß extrahierten Proteine Analysetechniken für Membranproteinkomplexe wie Blue Native Gelelektrophorese, Plasmonenresonanzspektroskopie und andere Techniken zur Analyse von Proteinkomplexen.

Die erfindungsgemäßen Verfahren eignen sich zur Extraktion von Membranproteinen aus biologischen Proben unter Erhalt der zellulären Grundstruktur der Proben, d.h. die Struktur der Membranproteine bleibt soweit wie möglich erhalten. Auf diese Weise lassen sich auch ganze Membranproteinkomplexe isolieren, die mit den herkömmlichen Methoden nicht oder nur schwer isolierbar sind. In der Regel lassen sich die erhaltenen Membranproteine mit geeigneten Antikörpern nachweisen

Soll das lineare amphipathische Kohlenhydrat-Polymer nach der Extraktion entfernt werden, so eignen sich dazu Zentrifugation, Filtration, magnetische Separation, Sedimentation, Chromatographie oder weitere dem Fachmann bekannte physikalische Separationsverfahren.

Das lineare amphipathische Kohlenhydrat-Polymer kann in dem erfindungsgemäßen Verfahren auch in immobilisierter Form, d.h. als Beschichtung von Oberflächen oder bevorzugt Partikeln eingesetzt werden. Insbesondere werden dabei magnetische Partikel bevorzugt, die mit dem linearen amphipathischen Kohlenhydrat-Polymer beschichtet sind. Beispielsweise können dies Magnetit oder Maghämit-Partikel mit Partikelgrößen zwischen 5 und 100 nm sein. Die Kohlenhydrat-Polymer-Beschichtung kann kovalent oder nicht-kovalent auf die Partikel aufgebracht sein. Beispielsweise werden die Partikel zunächst mit einer Verbindungsschicht aus z.B. Silica oder einem organischen Polymer belegt, auf die dann das lineare amphipathische Kohlenhydrat-Polymer aufgebracht wird.

Ebenfalls Gegenstand der vorliegenden Erfindung ist die Verwendung eines Kits zur Extraktion von Membranproteinen nach dem vorab beschriebenen erfindungsgemäßen Verfahren. Der Kit umfasst mindestens ein lineares amphipathisches Kohlenhydrat-Polymer und ein Lysemittel. Typischerweise enthält der Kit das lineare amphipathische Kohlenhydrat-Polymer
- in fester Form,
- in Form einer hochkonzentrierten Aufschlämmung des Kohlenhydrat-Polymers in Wasser, einem mit Wasser mischbaren organischen Lösungsmittel (z.B. Ethanol) oder einem wässrigen Puffersystem
- oder in Form einer wässrigen Suspension wie oben bei der Durchführung des erfindungsgemäßen Verfahrens beschrieben.

In der Regel enthält der Kit zusätzlich noch einen geeigneten Puffer, in dem das Kohlenhydrat-Polymer zur Extraktion in geeigneter Menge suspendiert werden kann. Weitere optionale Bestandteile sind z.B. Protease-Inhibitoren oder Waschpuffer. Geeignete Lysemittel wurden bereits beschrieben. Besonders bevorzugt wird Digitonin eingesetzt.

Mit dem erfindungsgemäßen Kit wird es dem Anwender ermöglicht, Membranproteine aus biologischen Proben auf einfache Weise zu extrahieren.

Die Verwendung des Kits zur Extraktion von insbesondere multiplen transmembranösen Proteinen aus biologischen Proben ist ebenfalls Gegenstand der vorliegenden Erfindung.

Auch ohne weitere Ausführungen wird davon ausgegangen, daß ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Die vollständige Offenbarung aller vor- und nachstehend aufgeführten Anmeldungen, Patente und Veröffentlichungen, insbesondere der korrespondierenden Anmeldung DE 10 2007 060599.6, eingereicht am 15.12.2007 ist durch Bezugnahme in diese Anmeldung eingeführt.

### Beispiel

### Erfindungsgemäße Extraktion von Membranproteinen aus HEK 293 Zellen

Waschpuffer: 1X PBS

### Protease Inhibitor Cocktail set III:

Calbiochem Art.Nr.: 539134
Extraktionspuffer I: 10mM PIPES pH 6.8
   0.02 Gew % Digitonin
   300mM Saccarose
   15mM NaCl
   0.5mM EDTA

Der Extraktionspuffer I kann optional eingesetzt werden, um vorab die cytosolischen Proteine zu extrahieren.
Extraktionspuffer II: 10mM PIPES pH 7.4
   300mM Saccarose
   15mM NaCl
   0.5mM EDTA

### Vorbereitung des Kohlenhydrat-Polymers (NVoy Polymer (Novexin, GB)

- 500 µL des Polymers (Suspension in Ethanol) in Eppendorf-Cup überführen
- 3 min bei 5.000g zentrifugieren
- Überstand verwerfen, Polymer mit 1000 µL Extraktionspuffer **II** waschen
- 3 min bei 5.000g zentrifugieren, Überstand verwerfen
- 2 mal wiederholen
- am Ende ca. 1:1 Slurry (50% Polymer/50% Puffer) herstellen
- Für die Extraktion daraus eine 2 %ige Lösung (Vol%) in Extraktionspuffer II herstellen

### Durchführung bei adherenten Zellen :

- T₇₅ Flaschen mit beispielweise HEK 293 Zellen (ca 90% bewachsen)
- Medium abgießen
- 2 x mit 10ml PBS (4°C) waschen, Waschpuffer verwerfen (zw. 0-37 °C.)
- + 10µl Protease Inhibitor Cocktail (optional)
- + 1000 µl 2,0 %ige (Vol.%)Lösung Polymer pro Flasche dazugeben, vorsichtig verteilen
- 30 min bei 4 °C stehen lassen (zwischen 10 und 60 min bzw.
   zwischen 0°C und 37°C)
- Zellen abschaben, 15 min bei 4°C, 16.000 g zentrifugieren

Dabei wird neben zellulären Kompartimenten auch das Polymer abzentrifugiert und aus der Probe entfernt.

=> der Überstand kann dann als Proteinfraktion der weiteren Analyse (z.B. der direkten massenspektrometrischen Untersuchung, sowie Analysentechniken von Membranproteinkomplexen (z.B. Blue Native Gelelektrophorese) und allen nativen enzymatischen wie immunologischen Analysemethoden) zugeführt werden

Die Durchführung des Verfahrens kann z.B. auch mit Suspensions-Zellen, isolierten Gewebszellen oder homogenisierten Geweben in gleicher Weise erfolgen.

## Patentansprüche

1. Verwendung von mindestens einem linearen amphipathischen Kohlenhydrat-Polymer zur Extraktion von Membranproteinen aus biologischen Proben.

2. Verwendung nach Anspruch 1, **dadurch gekennzeichnet, dass** zur Extraktion eine wässrige Suspension enthaltend zwischen 0,5 und 10 Gew% mindestens eines linearen amphipathischen Kohlenhydrat-Polymers eingesetzt wird.

3. Verwendung nach Anspruch 1 oder 2, **dadurch gekennzeichnet, dass** es sich bei den biologischen Proben um Gewebe, Zellen, Zellkulturen, Körperflüssigkeiten, Bakterien, Pilzen, Viren und/oder Pflanzen handelt.

4. Verfahren zur Extraktion von Membranproteinen aus biologischen Proben, **dadurch gekennzeichnet, dass** eine biologische Probe mit einer Mischung zumindest enthaltend ein oder mehrere lineare amphipathische Kohlenhydrat-Polymere versetzt wird.

5. Verfahren nach Anspruch 4, **dadurch gekennzeichnet, dass** die biologische Probe vor der Zugabe der Mischung zumindest enthaltend ein oder mehrere lineare amphipathische Kohlenhydrat-Polymere lysiert wird.

6. Verfahren nach einem der Ansprüche 4 oder 5, **dadurch gekennzeichnet, dass** die enthaltenen ein oder mehreren linearen amphipathischen Kohlenhydrat-Polymere ungeladen sind.

7. Verfahren nach einem oder mehreren der Ansprüche 4 bis 6, **dadurch gekennzeichnet, dass** die enthaltenen ein oder mehreren linearen amphipathischen Kohlenhydrat-Polymere aus Inulin oder Derivaten davon bestehen.

8. Verfahren nach einem oder mehreren der Ansprüche 4 bis 7, **dadurch gekennzeichnet, dass** die enthaltenen ein oder mehreren linearen amphipathischen Kohlenhydrat-Polymere ein lineares Poly-Fructose-Rückgrat aufweisen.

9. Verfahren nach einem oder mehreren der Ansprüche 4 bis 8, **dadurch gekennzeichnet, dass** die Extraktion bei Temperaturen zwischen 4 und 37°C erfolgt.

10. Verfahren nach einem oder mehreren der Ansprüche 4 bis 9, **dadurch gekennzeichnet, dass** die Konzentration der linearen amphipathischen Kohlenhydrat-Polymere in der Suspension oder Mischung zwischen 0.5 und 10 Gew.-% beträgt.

11. Verfahren nach einem oder mehreren der Ansprüche 4 bis 10, **dadurch gekennzeichnet, dass** die ein oder mehreren linearen amphipathischen Kohlenhydrat-Polymere nach der Extraktion mittels Zentrifugation, Filtration, magnetischer Separation, Sedimentation oder chromatographischer Methoden entfernt wird.

12. Verfahren nach einem oder mehreren der Ansprüche 4 bis 11, **dadurch gekennzeichnet, dass** die ein oder mehreren linearen amphipathischen Kohlenhydrat-Polymere auf magnetische Partikel aufgebracht sind.

13. Verwendung eines Kits zumindest enthaltend ein oder mehrere lineare amphipathische Kohlenhydrat-Polymere sowie mindestens ein Lysemittel ausgewählt aus der Gruppe der Detergenzien, oberflächenaktiven Substanzen und/oder Porenbildner zur Extraktion von Membranproteinen aus biologischen Proben.

## Claims

1. Use of at least one linear, amphipathic carbohydrate polymer for the extraction of membrane proteins from biological samples.

2. Use according to Claim 1, **characterised in that** an aqueous suspension comprising between 0.5 and 10% by weight of at least one linear, amphipathic carbohydrate polymer is employed for the extraction.

3. Use according to Claim 1 or 2, **characterised in that** the biological samples are tissues, cells, cell cultures, body fluids, bacteria, fungi, viruses and/or plants.

4. Method for the extraction of membrane proteins from biological samples, **characterised in that** a mixture at least comprising one or more linear, amphipathic carbohydrate polymers is added to a biological sample.

5. Method according to Claim 4, **characterised in that** the biological sample is lysed before the addition of the mixture at least comprising one or more linear, amphipathic carbohydrate polymers.

6. Method according to one of Claims 4 and 5, **characterised in that** the one or more linear, amphipathic carbohydrate polymers present are uncharged.

7. Method according to one or more of Claims 4 to 6, **characterised in that** the one or more linear, amphipathic carbohydrate polymers present consist of inulin or derivatives thereof.

8. Method according to one or more of Claims 4 to 7, **characterised in that** the one or more linear, amphipathic carbohydrate polymers present have a linear polyfructose backbone.

9. Method according to one or more of Claims 4 to 8, **characterised in that** the extraction is carried out at temperatures between 4 and 37°C.

10. Method according to one or more of Claims 4 to 9, **characterised in that** the concentration of the linear, amphipathic carbohydrate polymers in the suspension or mixture is between 0.5 and 10% by weight.

11. Method according to one or more of Claims 4 to 10, **characterised in that** the one or more linear, amphipathic carbohydrate polymers are removed by means of centrifugation, filtration, magnetic separation, sedimentation or chromatographic methods after the extraction.

12. Method according to one or more of Claims 4 to 11, **characterised in that** the one or more linear, amphipathic carbohydrate polymers are applied to magnetic particles.

13. Use of a kit at least comprising one or more linear, amphipathic carbohydrate polymers and at least one lysing agent selected from the group of the detergents, surface-active substances and/or pore formers for the extraction of membrane proteins from biological samples.

## Revendications

1. Utilisation d'au moins un polymère de carbohydrate amphipathique linéaire pour l'extraction de protéines de membrane à partir d'échantillons biologiques.

2. Utilisation selon la revendication 1, **caractérisée en ce qu'**une suspension aqueuse comprenant entre 0,5 et 10% en poids d'au moins un polymère de carbohydrate amphipathique linéaire est utilisée pour l'extraction.

3. Utilisation selon la revendication 1 ou 2, **caractérisée en ce que** les échantillons biologiques sont des tissus, des cellules, des cultures de cellules, des fluides du corps, des bactéries, des champignons, des virus et/ou des plantes.

4. Procédé pour l'extraction de protéines de membrane à partir d'échantillons biologiques, **caractérisé en ce qu'**un mélange comprenant au moins un ou plusieurs polymères de carbohydrate amphipathiques linéaires est ajouté à un échantillon biologique.

5. Procédé selon la revendication 4, **caractérisé en ce que** l'échantillon biologique est lysé avant l'ajout du mélange comprenant au moins un ou plusieurs polymères de carbohydrate amphipathiques linéaires.

6. Procédé selon l'une des revendications 4 et 5, **caractérisé en ce que** les un ou plusieurs polymères de carbohydrate amphipathiques linéaires présents sont non chargés.

7. Procédé selon une ou plusieurs des revendications 4 à 6, **caractérisé en ce que** les un ou plusieurs polymères de carbohydrate amphipathiques linéaires présents sont constitués par de l'inuline ou par ses dérivés.

8. Procédé selon une ou plusieurs des revendications 4 à 7, **caractérisé en ce que** les un ou plusieurs polymères de carbohydrate amphipathiques linéaires présents comportent une ossature en polyfructose linéaire.

9. Procédé selon une ou plusieurs des revendications 4 à 8, **caractérisé en ce que** l'extraction est mise en oeuvre à des températures entre 4 et 37°C.

10. Procédé selon une ou plusieurs des revendications 4 à 9, **caractérisé en ce que** la concentration des polymères de carbohydrate amphipathiques linéaires dans la suspension ou le mélange est entre 0,5 et 10% en poids.

11. Procédé selon une ou plusieurs des revendications 4 à 10, **caractérisé en ce que** les un ou plusieurs polymères de carbohydrate amphipathiques linéaires sont enlevés au moyen de procédés de centrifugation, de filtration, de séparation magnétique, de sédimentation ou de chromatographie après l'extraction.

12. Procédé selon une ou plusieurs des revendications 4 à 11, **caractérisé en ce que** les un ou plusieurs polymères de carbohydrate amphipathiques linéaires sont appliqués sur des particules magnétiques.

13. Utilisation d'un kit comprenant au moins un ou plusieurs polymères de carbohydrate amphipathiques linéaires et au moins un agent de lyse choisi parmi le groupe des détergents, des substances tensio-actives et/ou des agents de formation de pores pour l'extraction de protéines de membrane à partir d'échantillons biologiques.
